(19) Europäisches Patentamt
European Patent Office
Office européen des brevets

(11) Veröffentlichungsnummer: **0 650 731 A1**

(12) **EUROPÄISCHE PATENTANMELDUNG**

(21) Anmeldenummer: **94117049.0**

(22) Anmeldetag: **28.10.94**

(51) Int. Cl.6: **A61K 35/72**

(30) Priorität: **28.10.93 DE 4336888**

(43) Veröffentlichungstag der Anmeldung:
**03.05.95 Patentblatt 95/18**

(84) Benannte Vertragsstaaten:
**AT BE CH DE DK ES FR GB GR IE IT LI LU MC NL PT SE**

(71) Anmelder: **Thiemann Arzneimittel GmbH**
**Im Wirrigen 25**
**D-45731 Waltrop (DE)**
Anmelder: **Laboratoires BIOCODEX**
**19 rue Barbès**
**F-92126 Montrouge Cédex (FR)**

(72) Erfinder: **Friedland, Tilman**
**Am Kornfeld 3,**
**D-44534 Lünen (DE)**
Erfinder: **Seifert, Jürgen Prof.Dr. med**
**Karpfenteich 1,**
**D-24113 Kiel, (DE)**

(74) Vertreter: **Beszédes, Stephan G., Dr.**
**Patentanwalt,**
**Münchener Strasse 80a**
**D-85221 Dachau (DE)**

(54) **Produkt, erhältlich aus Hefezellen von Saccharomyces boulardii und dessen Verwendung.**

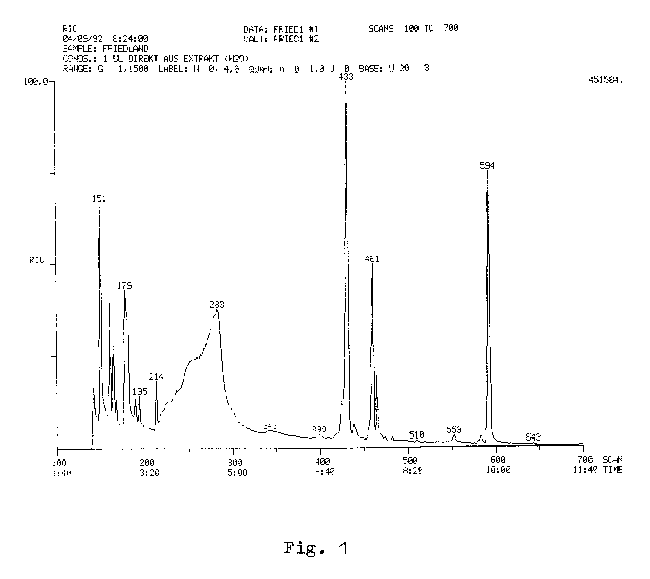

(57) Gegenstand der Erfindung ist ein Produkt mit dem Gaschromatogramm gemäß Fig. 1, erhältlich durch Inkubieren von Hefezellen von Saccharomyces boulardii bei 30 bis 38°C in einem wäßrigen Hefekultur-Medium, Abtrennen der Hefezellen, Konzentrieren des zellfreien Überstandes, dessen Extrahieren mit einem Alkanol mit 4 bis 8 Kohlenstoffatomen, Eindampfen der organischen Phase, Lösen des Rückstandes in Wasser, Dialyse der erhaltenen Lösung gegen destilliertes Wasser, Konzentrieren des Dialysates, Fällen der Proteine aus diesem und Abdampfen des Fällungsmittels.

Ferner ist Gegenstand der Erfindung die Verwendung des erfindungsgemäßen Produktes zur Herstellung von Antibiotika, Antimykotika, Immunsuppressiva und Antitumormitteln.

Fig. 1

Die Erfindung betrifft ein Produkt, erhältlich aus Hefezellen von Saccharomyces boulardii und dessen Verwendung.

Es ist bereits die Verwendung von Hefezellen von Saccharomyces boulardii gegen Amöbiase (EP-PS 195 870), zur Modifizierung der Darmflora (ER-OS 2 244 464 und FR-PS 3 501 M) und zur Verhütung oder Behandlung von Pseudomembrankolitis (EP-PS 149 579) bekannt. Solche Arzneimittelpräparate sind jedoch hinsichtlich der Aktivität noch nicht optimal.

Der Erfindung liegt daher die Aufgabe zugrunde, ein Produkt, erhältlich aus Hefezellen von Saccharomyces boulardii, welches eine stärkere Aktivität und ein breiteres Wirkungsspektrum hat, sowie seine Verwendung zu schaffen.

Das Obige wurde überraschenderweise durch die Erfindung erreicht.

Gegenstand der Erfindung ist ein Produkt mit dem Gaschromatogramm gemäß Fig. 1, erhältlich durch Inkubieren von Hefezellen von Saccharomyces boulardii bei 30 bis 38°C in einem wäßrigen Hefekultur-Medium, Abtrennen der Hefezellen, Konzentrieren des zellfreien Überstandes, dessen Extrahieren mit einem Alkanol mit 4 bis 8 Kohlenstoffatomen, Eindampfen der organischen Phase, Lösen des Rückstandes in Wasser, Dialyse der erhaltenen Lösung gegen destilliertes Wasser, Konzentrieren des Dialysates, Fällen der Proteine aus diesem und Abdampfen des Fällungsmittels.

Vorzugsweise wird das Inkubieren 3 bis 7 Tage lang durchgeführt.

Es ist auch bevorzugt, daß als Hefekultur-Nährmedium Sabouraud-Glukose mit einem pH-Wert von 6,5 verwendet wird.

Ferner ist es bevorzugt, daß das Abtrennen der Hefezellen durch Zentrifugieren durchgeführt wird. Zweckmäßig kann das Zentrifugieren mit 750 g/10 Minuten durchgeführt werden.

Weiterhin ist es bevorzugt, daß das Konzentrieren des zellfreien Überstandes auf $\frac{1}{20}$ bis $\frac{1}{40}$ durchgeführt wird.

Vorzugsweise wird das Konzentrieren durch Eindampfen zur Trockne und derauffolgendes Wiederaufnehmen in Wasser durchgeführt.

Es ist auch bevorzugt, daß das Extrahieren mit einem Butanol, insbesondere 1-Butanol, durchgeführt wird.

Ferner ist es bevorzugt, daß als Dialysemembran eine solche mit einer Porengröße, welche kleinere Moleküle als 1 000 D durchtreten läßt, verwendet wird.

Weiterhin ist es bevorzugt, daß die Proteine mit einem organischen Lösungsmittel gefällt werden. Vorzugsweise wird als organisches Lösungsmittel ein Keton oder Ketongemisch, insbesondere Aceton, verwendet.

Es ist auch bevorzugt, daß die Proteine durch Aussalzen gefällt werden.

Das erfindungsgemäße Produkt kann gereinigt worden sein. Es ist bevorzugt, daß eine Reinigung durch Chromatographie durchgeführt wird. Ferner ist es bevorzugt, daß als Chromatographie eine Säulenchromatographie, Dünnschichtchromatographie oder Hochdruckflüssigkeitschromatographie angewandt wird. Vorzugsweise wird zur Chromatographie als Eluiermittel ein Gemisch von Ethanol, Butanol und Wasser im Volumverhältnis von 3 : 4 : 0,5 oder/und danach oder vorher ein Gemisch von Acetonitril und Wasser im Volumverhältnis von 9 : 1 oder/und danach oder vorher ein Gemisch von Ethanol : Butanol : Wasser im Volumverhältnis von 3 : 3 : 1 verwendet. Soweit in dieser Beschreibung Ethanol steht, umfaßt es auch 96 gew.-%-iges und sonstiges konzentriertes wäßriges Ethanol.

Ferner ist Gegenstand der Erfindung das erfindungsgemäße Produkt zur Verwendung als Arzneimittel, insbesondere Antidiarrhoika, Antibiotika, Antimykotika, Antisuppressiva und Antitumormittel.

Gegenstand der Erfindung ist auch die Verwendung des erfindungsgemäßen Produktes zur Herstellung von Antidiarrhoika, Antibiotika, Antimykotika, Antisuppressiva und Antitumormitteln.

Das erfindungsgemäße Produkt hat nämlich gegenüber dem Stand der Technik überlegene pharmakologische Eigenschaften, insbesondere eine stärkere Aktivität und breiteres Wirkungsspektrum.

Hervorzuheben ist von der Verwendung zur Herstellung von Antidiarrhoika die zur Herstellung von solchen zur Vorbeugung und Behandlung von Sommer- und Reisedurchfall, Durchfall bei Antibiotika- und Strahlentherapie und Colitis.

Ein spezielles Beispiel für den Reisedurchfall ist die Amöbiase. Ein spezielles Beispiel für eine Diarrhoe bei Antibiotika- und Strahlentherapie, zu deren Behandlung die durch die erfindungsgemäße Verwendung erhältlichen Arzneimittel eingesetzt werden können, ist die Pseudomembrancolitis.

Die erfindungsgemäße Verwendung kann jedoch zur Herstellung von Antidiarrhoika zur Behandlung aller Arten von Diarrhoen erfolgen. Sie können nach ihrer Dauer in 4 Untertypen aufgeteilt werden.

a) Akute Diarrhoe, ein selbstlimitierendes Krankheitsgeschehen, das etwa 3 Tage anhält,

b) persistierende Diarrhoe, ein Diarrhoegeschehen, das deutlich länger als 3 Tage anhält,

c) chronische Diarrhoe, ein Krankheitsgeschehen, das in der Regel mehrere Wochen bis Monate andauert und

d) chronisch rezidivierende Diarrhoe, ein Krankheitsgeschehen, das über längere Zeit schubweise erfolgt, wobei die Diarrhoeschübe durch diarrhoefreie Zeiträume voneinander getrennt sind.

Speziell die Diarrhoeformen a) und d) lassen sich weiter je nach Auslöser in verschiedene Untertypen unterteilen; jeder dieser Untertypen ist als ein Spezialfall einer Diarrhoe anzusehen.

Eine akute Diarrhoe kann durch verschiedene Agenzien ausgelöst werden. An erster Stelle sind hier infektiöse Diarrhoen zu nennen. Die auslösenden Bakterien können unterschieden werden in nicht-invasive Bakterien, zum Beispiel Salmonellen und enterotoxische E. coli, und invasive Bakterien, zum Beispiel Shigellen, Yersinien, Campylobacten und invasive E. coli. Außerdem spielen Viren als Auslöser von akuten infektiösen Diarrhoen eine Rolle. Hier sind vor allem Rotaviren und parvovirenähnliche Organismen zu nennen. Auch Protozoen kommen als Auslöser dieser Diarrhoen in Frage, hier sind vor allen Gardia lamblia und Entamoeba histolytica zu nennen.

Akute Diarrhoen können aber auch unabhängig von lebenden Mikroorganismen hervorgerufen werden, so durch bakterielle Toxine, die mit der Nahrung aufgenommen werden, zum Beispiel Staphylococcus areus-Toxin, oder aber durch Arzneimittel, wie Antibiotika, Laxantien oder Zytostatika.

Weitere Auslöser von akuten Diarrhoen können Strahlentherapie (Bestrahlung des Abdomens), aber auch psychische Belastungen (psychogene Diarrhoen) und ebenso Intoxikationen des Magen-Darm-Traktes durch toxisch wirkende Verbindungen, zum Beispiel Alkohol, Arsen und Quecksilberverbindungen, sein.

Die chronischen Diarrhoen können durch verschiedene, teilweise kombinierte Faktoren ausgelöst werden. So kann ein hyperosmostischer Darminhalt zu Malabsorptions- bzw. Maldigestionsvorgängen im Darm führen, die letztlich eine erhöhte Stuhlfrequenz zur Folge haben. Daneben kann der Darm zur Hypersekretion angeregt werden, indem spezifische oder unspezifische Entzündungen im Darm stattfinden, beziehungsweise Enterotoxine oder auch Tumorerkrankungen die Sekretionsverhältnisse nachhaltig ändern. Außerdem kann der natürliche Fluß des Chymus (Nahrungsbrei) durch Veränderung der Darmmotilität, zum Beispiel peristaltische Bewegung, gestört werden, so daß auch dieses zu Diarrhoen führt. Ursachen hierfür können sein, Endokrinopathien, zum Beispiel Enzymmangelsituationen oder ein Diabetes mellitus sowie auch eine bakterielle Überwucherung (infektiöse chronische Diarrhoe). Ist das gesamte funktionelle Geschehen im Magen-Darm-Trakt gestört (Reizdarmsyndrom), so kann dieses ebenfalls mit dem Symptom Diarrhoe einhergehen. Wie auch bei der akuten Diarrhoe kann auch bei der chronischen Diarrhoe eine medikamentöse Behandlung Ursache des Symptoms sein. Auch hier sind Wirkstoffe, wie Antibiotika, Laxantien und Zytostatika, zu nennen, darüber hinaus aber beispielsweise auch Analgetika, Antirheumatika und Antazida. Neben diesen genannten Auslösefakturen für eine chronische Diarrhoe gibt es verschiedene weitere wie zum Beispiel die Krankheitsbilder Morbus Crohn und Colitis ulcerosa (chronisch entzündliche Darmerkrankung, deren Auslösefaktoren heute noch nicht endgültig geklärt sind). Ferner können Ischämie, kollagene Colitis, Divertikulose aber auch Nahrungsmittelallergien und, wie auch bei der akuten Diarrhoen, abdominelle Bestrahlungen zu chronischen Diarrhoen führen.

Das erfindungsgemäße Produkt kann in Form von Arzneimittelpräparaten, zweckmäßig in Mischung mit 1 oder mehr in der Pharmazie üblichen Träger- und/oder Hilfsstoff(en), vorliegen. Bevorzugt sind Arzneimittelpräparate zur oralen Anwendung.

Zur Untersuchung der pharmakologischen Wirksamkeit eines erfindungsgemäßen Produktes wurden die folgenden Versuche durchgeführt.

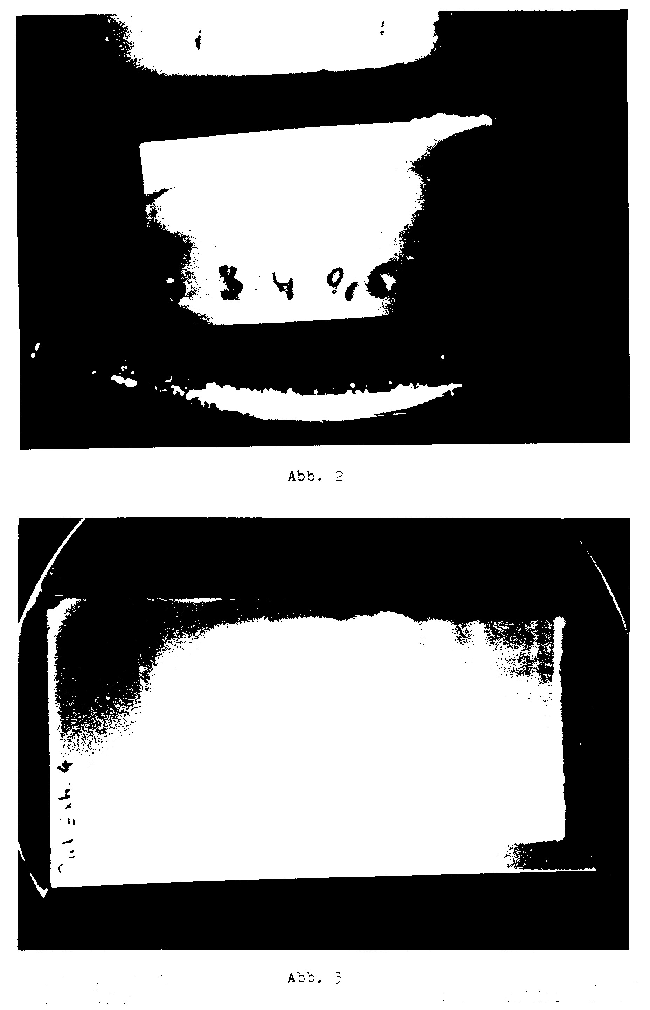

Nachdem der Hefekultur-Überstand konzentriert ($\frac{1}{20}$ des Ausgangsvolumens), extrahiert (2-mal mit 1 Butanol), dialysiert (Abtrennung der Moleküle mit einem Molekulargewicht < 1000 D) und von Proteinen befreit wurde, wurde der so erhaltene Extrakt einem Biotest unterzogen. Mit Hilfe einer Glaskapillare wurden 3 Portionen auf dieselbe Bande (etwa 25 µl Extrakt) auf eine Dünnschichtchromatographie(DC)-Platte strichförmig aufgetragen. Der Ausdruck "3 Portionen auf dieselbe Bande" bedeutet, daß die erste Portion der zu chromatographierenden Lösung strichförmig aufgetragen und anschließend die DC-Platte wie angegeben getrocknet wurde und, als das Lösungsmittel der ersten Portion vollständig verdampft war, auf den Auftragebereich (strichförmig) der ersten Portion die zweite Portion aufgetragen wurde, und nachdem diese wie beschrieben wiederum eingetrocknet war, mit der dritten Portion identisch verfahren wurde. Die Chromatographieplatte bestand aus einer Aluminiumfolie, beschichtet mit Kieselgel 60, sie hatte eine Abmessung von etwa 2 x 4 cm. Die mit einer Heizlampe bei 72ºC getrocknete DC-Folie wurde auf eine Agarplatte gelegt, festgedrückt und mit einer noch flüssigen Weichagar-E. coli 0 157-Suspension überschichtet (Sabouraud-Glukose-Agar, pH-Wert = 6,5; 5,5 Gew.-% Agar-Agar-Anteil mit einer Temperatur von etwa 43ºC und darin suspendierten E. coli-Bakterien). Nach dem Erstarren des sogenannten Overlayer-Agars wurde über Nacht im Brutschrank bei 37ºC inkubiert. Das Ergebnis nach der Inkubationszeit zeigt Abb. 2. Man sieht einen trüben Bereich auf der Agarplatte, dort sind E. coli-Bakterien gewachsen, oval über der weißen DC-Folie erkennt man einen klaren Agar-Bereich, eine sogenannte Hemmzone, innerhalb derer sich die Bakterien nicht vermehren konnten.

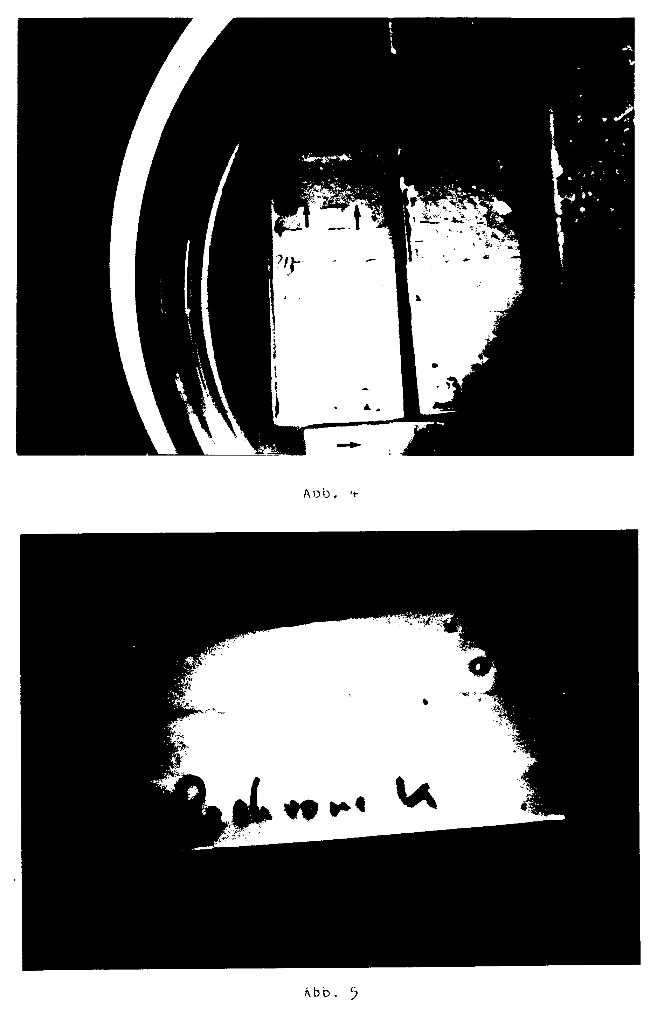

Ein solchermaßen sich als antibiotisch wirksam erwiesener Extrakt, zu dessen Herstellung keine Chromatographie angewandt wurde, wurde zur Trennung von Bestandteilen einer Dünnschichtchromatographie unterworfen. Hierzu wurden auf eine DC-Platte mit Abmessungen von 20 x 20 cm 150 bis 200 $\mu$l der wie vorstehend beschrieben erhaltenen Probelösung strichförmig aufgetragen. Nachdem die DC-Platte getrocknet war, wurde sie in einer Chromatographiekammer entwickelt, wobei als Eluiermittel ein Gemisch aus Ethanol, Butanol und Wasser im Volumverhältnis von 3 : 4 : 0,5 verwendet wurde. Nachdem in der aufsteigenden Chromatographie das Laufmittel eine Höhe von 6 cm erreicht hatte, wurde die Entwicklung abgebrochen, die DC-Platte aus der Kammer entnommen und getrocknet (vgl. Abb. 3). Unter UV-Licht von einer Wellenlänge von 365 nm wurden die fluores-zierenden Banden des Dünnschichtchromatogram-mes mit Bleistift markiert und numeriert. Anschließend wurde ein kleiner Teil des Chromatogrammes abgeschnitten (Schnittrichtung in Entwicklungsrichtung) und dem oben genannten Biotest unterzogen. Nach Übernacht-Inkubation zeigte sich, daß oberhalb einer Bande des Chromatogrammes sich die Bakterien nicht vermehren konnten, was auf die antibiolisch aktivste Wirksubstanz hinweist. Anhand von Bleistiftmarkierung und Numerierung ließ sich aus dem nicht dem Biotest unterworfenen Chromatogramm die entsprechende Bande auffinden. Das Ergebnis des Biotestes eines entwickelten Chromatogrammes zeigt Abb. 4. Hier wurden im Vergleich verschiedene Chromatogramme dem Biotest unterworfen, sie unterschieden sich durch die verschieden langen Entwicklungsstrecken. Wie auf dem Bild zu erkennen ist (Pfeile), befindet sich jeweils oberhalb der Bande, die mit 7 beziffert wurde, die Hemmzone.

Der Bereich der den antibiotisch aktivsten Bestandteil enthielt (Chromatographie-Bande Nr. 7, $R_f$-Wert = 0,64 cm) wurde mit Hilfe eines Skalpells aus den Chromatogramm herausgekratzt und mit den oben genannten Eluiermittel (20 ml) versetzt. Nach kräftigem Schütteln und Zentrifugieren wurde das Eluiermittel dekantiert; dieser Vorgang wurde noch 4-mal wiederholt. Nach Eindampfen des Eluiermittels und Wiederaufnahme des Rückstandes in 200 ml destillierten Wassers wurden 25 $\mu$l hiervon dem Biotest unterworfen. Das Ergebnis zeigt Abb. 5. Hier zeigt sich, ähnlich wie in Abb. 2, ein ovaler Hemmbereich, innerhalb dessen die Vermehrung der Bakterien verhindert wurde.

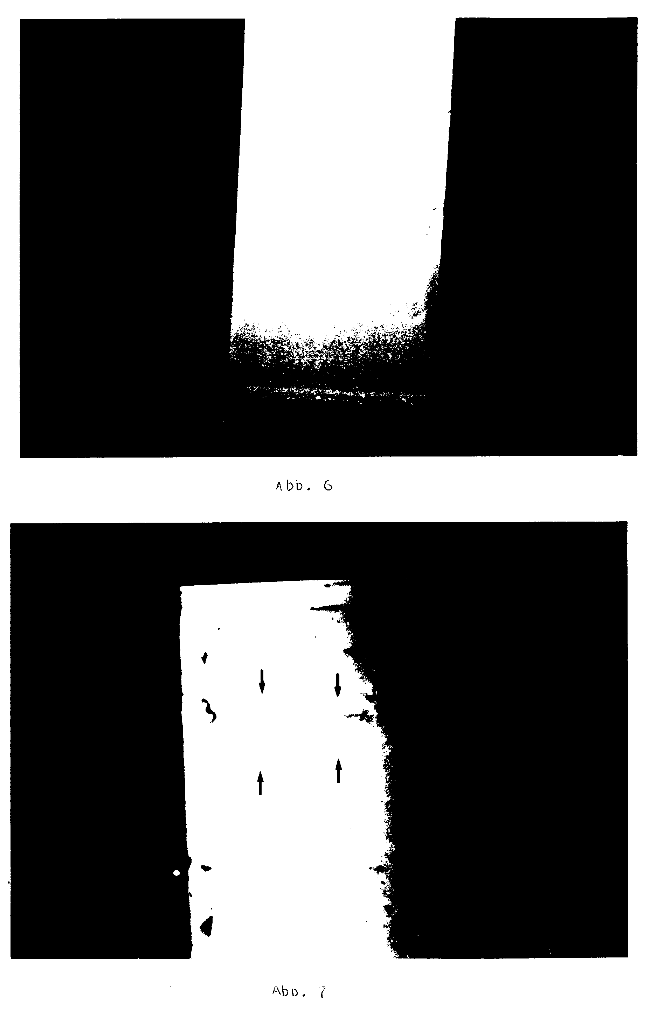

Nachdem sich auf diese Weise die eluierte Fraktion des ersten DC-Laufes als wirksam erwiesen hat, wurde diese einer erneuten Fraktionierung mit Hilfe der DC unterworfen. Hierbei wird wie oben angegeben verfahren, es wurde jedoch als Eluiermittel ein Gemisch aus Acetonitril und Wasser im Volumverhältnis von 9 : 1 verwendet. Auch beim zweiten DC-Lauf wurde nach 6 cm Laufstrecke abgebrochen, das Chromatogramm getrocknet und unter UV-Licht betrachtet. Die fluoreszierenden Banden wurden wiederum mit Bleistift markiert und numeriert, ein Teil des Chromatogrammes wurde abgetrennt und dem Biotest unterworfen. Die Abb. 6 und 7 zeigen das Ergebnis dieses Versuches. In Abb. 6 erkennt man das Rechromatogramm, bei dem die Anzahl der Banden gegenüber dem ersten DC-Lauf verringert sind. Der $R_f$-Wert des antibiotisch wirksamsten Bestandteiles betrug 0,73. In Abb. 7 markieren wiederum Pfeile die Hemmzone, die durch die Wirksubstanz im Chromatogramm hervorgerufen wurde.

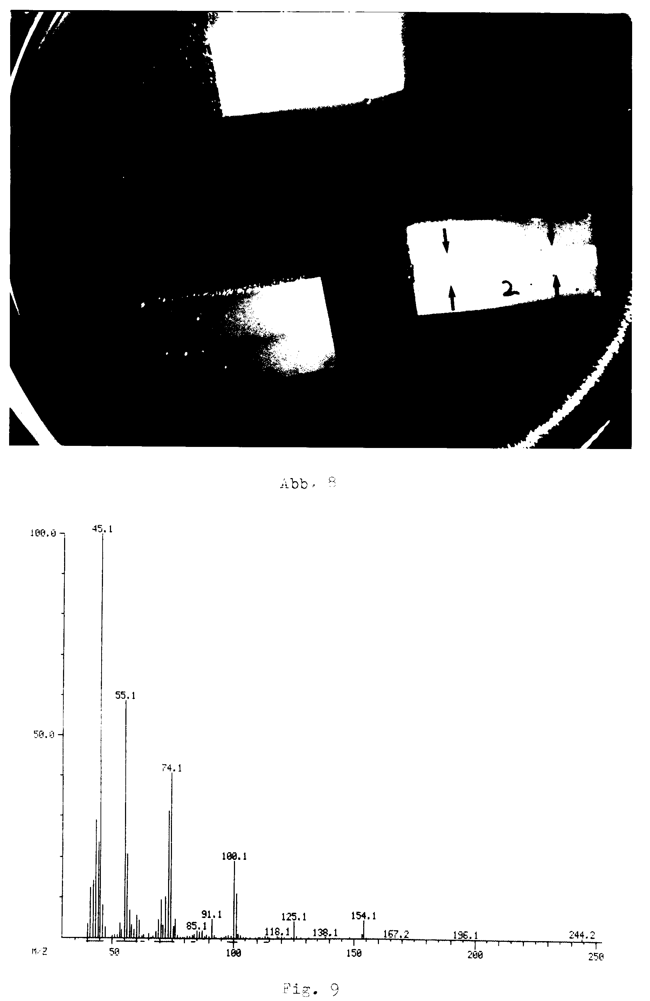

Das Eluieren der betreffenden Bande erfolgte parallel zu der oben angegebenen Weise, es wurde hier allerdings jeweils 5-mal mit beiden Eluiermitteln eluiert (Ethanol-Butanol-Wasser-Gemisch und Acetonitril-Wasser-Gemisch). Zur sicheren Identifizierung der fraglichen Bande wurden die in Abb. 6 mit 1, 2 bzw. 3 markierten Banden getrennt eluiert und dem Biotest unterzogen. Das Ergebnis zeigt Abb. 8. Hier sind die entsprechenden Eluate mit Hilfe des Biotests auf ihre Wirksamkeit untersucht worden. Deutlich zeigt sich, daß die mit 2 bezeichnete Probe (Pfeile) eine Hemmzone hervorrief.

Diese wirksame Fraktion des Saccharomyces boulardii-Kulturüberstandes wurde zur weiteren Identifikation der Wirksubstanz mit Hilfe der Gaschromatographie (GC) weiter fraktioniert. Das hierfür verwandte GC-Gerät arbeitet computergestützt und vollautomatisch. Es handelte sich hierbei um ein Gerät der Firma Hewlett Packard, ausgerüstet mit einer 12 m langen HP-Ultra 1-Säule mit einem Innendurchmesser von 0,2 cm. Diese Säule ist belegt mit einem 0,33 $\mu$m starken Methylsilicon-Cross-Link-Belegungsfilm. Als Trägergas wurde Helium verwendet. Zur gaschromatographischen Auftrennung des wie oben beschrieben erhaltenen Gemisches wurde ein Temperaturprogramm von 70°C bis 280°C mit einer Gradientensteilheit von 20°C pro Minute gefahren. Das Gerät ist mit einem Splitlessinjektor ausgestattet.

Das Gaschromatogramm ist in Fig 1 dargestellt. Wie Fig. 1 eindeutig zeigt, enthält die obige Fraktion mehrere Substanzen. Die meisten dieser Substanzen liegen jedoch in kleiner Konzentration vor (als Maß für die Konzentration dient die Fläche unter der Kurve). Der zwischen 3,20 und 5 Minuten liegende Peak stellt den Hauptteil der Probelösung dar. Das zu diesem Peak gehörende Massenspektrum ist in Fig. 9 dargestellt. Diese Substanz kann durch 2- bis 3-maliges Wiederholen der Chromatographierarbeitsgänge, also insgesamt 4- bis 5 Chromatographiearbeitsgänge gewonnen werden.

Die Erfindung wird an Hand des folgenden Beispieles näher erläutert.

Beispiel

a) Anzucht, Kultur, Kulturüberstand und Konzentrieren

10 Kapseln lyophilisierter Zellen der Hefe Saccharomyces boulardii (Präparat Perenterol®), entsprechend 0,5 g, wurden in 3 ml steriler Natriumchloridlösung suspendiert und für 3 bis 5 Minuten darin belassen. Diese Hefezellsuspension wurde anschließend in 50 ml Kulturmedium gegeben (Sabouraud-Glukose-Medium) und in einem 100 ml Weithals-Erlenmeyer-Kolben bei 37°C inkubiert. Nach 24 Stunden wurden 4 x 1 ml auf 50 ml frischen Mediums überimpft (2%-ige Beimpfung). Dieser Kulturansatz wurde für 5 Tage bei 37°C inkubiert. Anschließend wurde bei 750 g für 10 Minuten zentrifugiert. Das Sediment, bestehend aus Hefezellen, wurde verworfen, während der zellfreie Überstand weiterverarbeitet wurde. Mit Hilfe eines Rotationsverdampfers wurde der Kulturüberstand zur Trockne eingedampft und der Rückstand wurde mit Wasser auf 10 ml aufgefüllt.

b) Extraktion

10 ml des konzentrierten Saccharomyces boulardii-Kulturüberstandes wurden mit 20 ml 1-Butanol versetzt und 20 Minuten lang gerührt. Die organische Phase wurde dekantiert und der Vorgang wurde wiederholt. Die wäßrige Phase wurde verworfen, während die 20 ml organische Phase mit den Rotationsverdampfer zur Trockne eingedampft wurden. Der Rückstand wurde mit Wasser auf 10 ml aufgefüllt.

c) Dialyse und Proteinfällung

Je 5 ml des 1-Butanol-Extraktes wurden in einen Dialyseschlauch eingeschlossen, dessen Membran Moleküle mit einem Molekulargewicht < 1000 D durchläßt, während sie Moleküle mit einem Molekulargewicht > 1000 D zurückhält. Die Dialyse erfolgte 2-mal, wobei jeweils gegen 100 ml destillierten Wassers unter Rühren über Nacht dialysiert wurde. Die 200 ml Dialysat wurden mit dem Rotationsverdampfer zur Trockne eingedampft. Der Rückstand wurde mit Wasser auf 5 ml aufgefüllt. Der Inhalt des Dialyseschlauches wurde verworfen.

Die 5 ml konzentrierten, extrahierten und dialysierten Kulturüberstandes von Saccaromyces boulardii wurden so lange mit Aceton versetzt, bis eine vollständige Proteinausfällung stattgefunden hatte. Der entstandene Proteinniederschlag wurde mittels Zentrifugierens bei 4 500 g sedimentiert und verworfen. Das Zentrifugat wurde mit dem Rotationsverdampfer zur Trockne eingedampft und der Rückstand mit Wasser auf 5 ml aufgefüllt.

d) Chromatographie

Je 1 ml der erhaltenen Lösung wurde strichförmig auf eine Dünnschichtchromatographieplatte aufgetragen. Bei der Platte handelte es sich um 20 x 20 cm große mit Kieselgel 60 beschichtete Aluminiumfolien. Nachdem die Probe vollständig getrocknet war, wurde das Chromatogramm in einem Eluiermittel, bestehend aus Acetonitril und Wasser im Volumverhältnis (v/v) von 9 : 1 entwickelt. Sobald die mobile Phase (das Eluiermittel) eine Höhe von 6 cm erreicht hatte, wurde die Entwicklung der Chromatogramme abgebrochen, die Chromatographieplatten wurden den Chromatographiekammern entnommen und getrocknet. Unter UV-Licht von 365 nm sind die entstandenen Banden erkennbar. Der $R_f$-Wert der betreffenden antibiotisch wirksamen Bande beträgt 0,64 cm. Diese Chromatographiebande wurde aus dem Chromatogramm herausgekratzt, das die Wirksubstanz enthaltende Kieselgel 60-Material wurde gesammelt und mit 20 ml Eluiermittel versetzt und kräftig geschüttelt. Nachdem sich die Feststoffe abgesetzt hatten, wurde das Eluiermittel dekantiert. Dieser Vorgang wurde noch 4-mal wiederholt. Die erhaltene Lösung wurde mit einem Rotationsverdampfer zur Trockne eingedampft und mit Wasser auf 5 ml aufgefüllt. Je 1 ml dieser Lösung wurde erneut auf eine mit Kieselgel 60-beschichtete Dünnschichtchromatographieplatte mit Abmessungen von 20 x 20 cm strichförmig aufgetragen. Nachdem die Proben getrocknet waren, wurden die Chromatogramme in der Chromatographiekammer entwickelt, wobei als Eluiermittel eine Mischung aus Ethanol (96 gew.-%-ig), Butanol und Wasser im Volumverhältnis (v/v/v) von 3 : 3 : 1 verwendet wurde. Wie beim ersten Chromatographielauf wurde nach 6 cm Entwicklungsstrecke das Chromatogramm entnommen und getrocknet. Die antibiotisch aktive Bande weist einen $R_f$-Wert von 0,73 cm auf. Diese Bande wurde aus den Chromatogrammen herausgekratzt und das die Wirksubstanz enthaltende Kieselgel 60 wurde gesammelt und mit 20 ml einer Mischung aus Acetonitril und Wasser im Volumverhältnis (v/v) von 9 : 1 versetzt. Nachdem sich die Feststoffe abgesetzt hatten, wurde der klare Überstand dekantiert und der Vorgang wurde 4-mal wiederholt. Hieran anschließend wurde das Sediment mit 20 ml eines Gemisches, bestehend aus Ethanol (96 gew.-%-ig), Butanol und Wasser im Volumverhältnis (v/v/v) von 3 : 3 : 1, versetzt und kräftig geschüttelt. Nach Absetzen der Feststoffe wurde der klare Überstand dekantiert und der Vorgang wurde noch 4-mal wiederholt. Sämtliche flüssigen Phasen, insgesamt 200 ml, wurden im Rotationsverdampfer zur Trockne eingedampft und mit Wasser auf 5 ml aufgefüllt. Die erhaltene Lösung war eine weitgehende Anreicherung einer antibiotisch aktiven Fraktion aus dem Saccharomyces boulardii-Kulturüberstand.

**Patentansprüche**

1. Produkt mit dem Gaschromatogramm gemäß Fig. 1, erhältlich durch Inkubieren von Hefezellen von Saccharomyces boulardii bei 30 bis 38°C in einem wäßrigen Hefekultur-Medium, Abtrennen der Hefezellen, Konzentrieren des zellfreien Überstandes, dessen Extrahieren mit einem Alkanol mit 4 bis 8 Kohlenstoffatomen, Eindampfen der organischen Phase, Lösen des Rückstandes in Wasser, Dialyse der erhaltenen Lösung gegen destilliertes Wasser, Konzentrieren des Dialysates, Fällen der Proteine aus diesem und Abdampfen des Fällungsmittels.

2. Produkt nach Anspruch 1, dadurch gekennzeichnet, daß das Inkubieren 3 bis 7 Tage lang durchgeführt wird.

3. Produkt nach Anspruch 1 oder 2, dadurch gekennzeichnet, daß als Hefekultur-Nährmedium Sabouraud-Glukose mit einem pH-Wert von 6,5 verwendet wird.

4. Produkt nach Anspruch 1 bis 3, dadurch gekennzeichnet, daß das Abtrennen der Hefezellen durch Zentrifugieren durchgeführt wird.

5. Produkt nach Anspruch 1 bis 4, dadurch gekennzeichnet, daß das Konzentrieren des zell-freien Überstandes auf $\frac{1}{20}$ bis $\frac{1}{40}$ durchgeführt wird.

6. Produkt nach Anspruch 1 bis 4, dadurch gekennzeichnet, daß das Konzentrieren durch Eindampfen zur Trockne und darauffolgendes Wiederaufnehmen in Wasser durchgeführt wird.

7. Produkt nach Anspruch 1 bis 6, dadurch gekennzeichnet, daß das Extrahieren mit einem Butanol, insbesondere 1-Butanol, durchgeführt wird.

8. Produkt nach Anspruch 1 bis 7, dadurch gekennzeichnet, daß als Dialysemembran eine solche mit einer Porengröße, welche kleinere Moleküle als 1 000 D durchtreten läßt, verwendet wird.

9. Produkt nach Anspruch 1 bis 8, dadurch gekennzeichnet, daß die Proteine mit einem organischen Lösungsmittel gefällt werden.

10. Produkt nach Anspruch 9, dadurch gekennzeichnet, daß als organisches Lösungsmittel ein Keton oder Ketongemisch, insbesondere Aceton, verwendet wird.

11. Produkt nach Anspruch 1 bis 8, dadurch gekennzeichnet, daß die Proteine durch Aussalzen gefällt werden.

12. Produkt nach Anspruch 1 bis 11, dadurch gekennzeichnet, daß eine Reinigung durch Chromatographie durchgeführt wird.

13. Produkt nach Anspruch 1 bis 12, dadurch gekennzeichnet, daß als Chromatographie eine Säulenchromatographie, Dünnschichtchromato-graphie oder Hochdruckflüssigkeitschromato-graphie angewandt wird.

14. Produkt nach Anspruch 1 bis 13, dadurch gekennzeichnet, daß zur Chromatographie als Eluiermittel ein Gemisch von Ethanol, Butanol und Wasser im Volumverhältnis von 3 : 4 : 0,5 oder/und danach oder vorher ein Gemisch von Acetonitril und Wasser im Volumverhältnis von 9 : 1 oder/und danach oder vorher ein Gemisch von Ethanol : : Butanol : Wasser im Volumverhältnis von 3 : 3 : 1 verwendet wird.

15. Produkt nach Anspruch 1 bis 14 zur Verwendung als Arzneimittel.

16. Verwendung des Produktes nach Anspruch 1 bis 15 zur Herstellung von Antidiarrhoika, Antibiotika, Antimykotika, Immunsuppressiva und Antitumormitteln.

17. Verwendung nach Anspruch 16, dadurch gekennzeichnet, daß die Verwendung zur Herstellung von Antidiarrhoika eine solche zur Herstellung von solchen zur Vorbeugung und Behandlung von Sommer- und Reisedurchfall, Durchfall bei Antibiotika- und Strahlentherapie und Colitis ist.

Fig. 1

Abb. 2

Abb. 3

Abb. 4

Abb. 5

Abb. 6

Abb. 7

Abb. 8

Fig. 9

| | EINSCHLÄGIGE DOKUMENTE | | EP 94117049.0 | |
|---|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl.6) | |
| D,A | EP - A - 0 195 870 (LABORATOIRES BIOCODEX) * Ansprüche 1-6 * -- | 1-17 | A 61 K 35/72 | |
| D,A | EP - A - 0 149 579 (LABORATOIRES BIOCODEX) * Ansprüche 1-5 * ---- | 1-17 | | |
| | | | RECHERCHIERTE SACHGEBIETE (Int. Cl.6) | |
| | | | A 61 K 35/00 | |

Der vorliegende Recherchenbericht wurde für alle Patentansprüche erstellt.

| Recherchenort | Abschlußdatum der Recherche | Prüfer |
|---|---|---|
| WIEN | 30-12-1994 | BRUS |